(19) **Europäisches Patentamt / European Patent Office / Office européen des brevets**

(11) **EP 4 274 486 B1**

(12) **FASCICULE DE BREVET EUROPEEN**

(45) Date de publication et mention de la délivrance du brevet:
**22.05.2024 Bulletin 2024/21**

(21) Numéro de dépôt: **22710655.6**

(22) Date de dépôt: **18.02.2022**

(51) Classification Internationale des Brevets (IPC):
***A61B 10/00*** *(2006.01)*

(52) Classification Coopérative des Brevets (CPC):
**A61B 10/0012; A61B 10/007;** A61B 2010/0019

(86) Numéro de dépôt international:
**PCT/FR2022/050293**

(87) Numéro de publication internationale:
**WO 2022/180328 (01.09.2022 Gazette 2022/35)**

(54) **DISPOSITIF D'ANALYSE D'URINE POUR MESURER LA TEMPÉRATURE DE L'URINE**

URINANALYSEGERÄT ZUR MESSUNG DER TEMPERATUR DES URINS

URINE ANALYSIS DEVICE FOR MEASURING THE TEMPERATURE OF THE URINE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.02.2021 FR 2101762**

(43) Date de publication de la demande:
**15.11.2023 Bulletin 2023/46**

(73) Titulaire: **Withings
92130 Issy les Moulineaux (FR)**

(72) Inventeurs:
• **BEDETTI, Thomas
  92130 Issy les Moulineaux (FR)**
• **OUESLATI, Ianis
  92130 Issy les Moulineaux (FR)**
• **RAFIK, Walid
  92130 Issy les Moulineaux (FR)**
• **AIMON, Nicolas
  92130 Issy les Moulineaux (FR)**
• **DEWAVRIN, Julius
  92130 Issy les Moulineaux (FR)**

(74) Mandataire: **Withings IP
2, rue Maurice Hartmann
92130 Issy-les-Moulineaux (FR)**

(56) Documents cités:
WO-A1-90/13797    WO-A1-2005/092177
WO-A1-2020/221943    WO-A2-2004/036343
JP-A- S63 176 536    JP-A- S63 189 537

## Description

### Domaine technique

La présente divulgation relève du domaine des dispositifs d'analyse d'urine. Plus précisément, la présente divulgation relève d'un dispositif d'analyse d'urine permettant de déterminer la température de l'urine. La présente divulgation vise également un procédé de mesure de la température de l'urine.

### Technique antérieure

La mesure de la température basale corporelle (BBT, de l'anglais *Basal Body Température)* est une méthode couramment utilisée par des femmes souhaitant déterminer leur période de fertilité.

La méthode est basée sur le principe que la température basale corporelle d'une femme fertile suit un schéma bi-phasique. La première phase correspond à la phase folliculaire du cycle menstruel, dans laquelle la température basale sera relativement basse. La deuxième phase correspond à la phase lutéale, qui commence après l'ovulation, dans laquelle une augmentation de la température basale peut être observée.

Les femmes utilisant cette méthode sont classiquement censées mesurer leur température orale, vaginale ou rectale tous les jours au réveil. Cependant, cette méthode apparaît intrusive. Il peut également être incommode pour beaucoup de femmes de mesurer leur température tous les jours.

Ainsi, il a été proposé des dispositifs de mesure de température à partir de l'urine. Ces dispositifs sont à fixer ou à monter dans les toilettes pour analyser l'urine sécrétée par une utilisatrice.

Dans certains cas, un échantillon d'urine peut être récolté durant la miction pour être canalisé vers un capteur de température. Néanmoins, ce genre de dispositif peut être difficile à installer, requérant par exemple d'être intégré aux toilettes. En outre, ce genre de dispositif apparaît peu pratique à utiliser, l'utilisatrice devant s'assurer qu'un échantillon suffisant d'urine est récolté lors de la miction.

Dans d'autres cas, la température de l'urine peut être mesurée à distance, par exemple à l'aide de capteur infrarouge. S'il n'est plus nécessaire de récolter un échantillon d'urine, la précision de la mesure de température semble insuffisante pour détecter des variations de la température basale.

La présente divulgation vise à proposer un dispositif d'analyse d'urine pour mesurer la température de l'urine ne présentant pas au moins certains des inconvénients présentés ci-dessus.

Le document JPS63176536 se rapporte à un thermomètre, dans une cuvette de toilettes,

qui mesure une température corporelle en détectant une température de l'urine, et qui correspond à un dispositif selon le préambule de la revendication 1.

Le document WO2005/092177 décrit un module avec un capteur de température ambiante et de peau. Le module peut être placée dans une couche, pour être au contact de l'urine.

### Résumé

L'invention est définie par les revendications jointes à la fin de la description.

Il est notamment proposé un dispositif d'analyse d'urine configuré pour être entièrement positionné à l'intérieur de toilettes, le dispositif comprenant :

- un boîtier,
- une plaque conductrice de chaleur, montée sur le boîtier, la plaque conductrice de chaleur comprenant une face libre destinée à recevoir un jet d'urine directement d'un utilisateur urinant dans les toilettes, et
- une pluralité de capteurs de température, chaque capteur de température étant configuré pour mesurer localement une information de température de la plaque conductrice.

Ainsi, le dispositif peut prendre une mesure précise de la température de l'urine, notamment pour détecter une période de fécondité ou un épisode fiévreux. La plaque conductrice peut entrer au contact d'un jet d'urine sans que l'utilisateur ne se préoccupe du dispositif. A l'aide de la diffusion de la chaleur au sein de la plaque conductrice, la plaque conductrice peut agir comme un filtre spatial permettant d'homogénéiser la température de l'urine sur toute la surface de la plaque. Le ou les capteur(s) de température peuvent relever la température de la plaque conductrice quel que soit le point d'impact du jet d'urine sur la plaque conductrice. La température peut être relevée même si le point d'impact n'est pas stable. Par ailleurs, le dispositif, positionné dans les toilettes, peut être discret. Le dispositif de mesure d'urine est configuré pour, à partir de la mesure de la température de l'urine, générer une donnée de température qui peut être transmise par des moyens d'une communication sans-fil.

Les caractéristiques exposées dans les paragraphes suivants peuvent, optionnellement, être mises en oeuvre. Elles peuvent être mises en oeuvre indépendamment les unes des autres ou en combinaison les unes avec les autres.

L'information de température peut notamment être une valeur de température ou une évolution de la valeur de température dans le temps, par exemple au cours d'une miction, c'est-à-dire la température de l'urine au cours du temps. L'information de température peut être mesurée via une grandeur physique du capteur de température, comme une tension à ses bornes. Les mesures de température peuvent être obtenues de façon discrète, quasi-continues ou continues.

Le ou les capteurs de température peuvent être reçus ou positionné dans le boîtier. En particulier, le boîtier et la plaque conductrice de chaleur peuvent définir un volume à l'intérieur duquel se trouvent le ou les capteurs de température. Pour éviter que les capteurs de température ne soient au contact du jet d'urine, le volume peut être étanche au jet d'urine.

La plaque conductrice peut avoir une surface supérieure à 3 500 mm$^2$, de voire supérieure à 3 800 mm$^2$. Ainsi, la surface disponible pour entrer au contact du jet d'urine est conséquente et confortable. La probabilité pour que le jet d'urine entre au contact de la plaque conductrice est augmentée.

La plaque conductrice peut être en un métal conducteur de chaleur. Ainsi, la plaque conductrice a une bonne conductivité. Une amélioration de la conductivité peut permettre une réduction du nombre de capteurs de température mesurant la température de la plaque conductrice.

La plaque conductrice peut notamment être en aluminium, de sorte à avoir une bonne résistance à l'eau et l'humidité des toilettes. La plaque conductrice peut ainsi conserver une bonne esthétique. Alternativement, la plaque conductrice pourrait être un alliage de cuivre ou un acier inoxydable.

Alternativement, la plaque conductrice peut être en un plastique conducteur de chaleur, ce qui permet à la fois une bonne conductivité et une simplification de la fabrication. Un plastique conducteur de chaleur peut être un polymère chargé avec des éléments conducteurs comme des fibres de graphite ou des céramiques (nitrure d'aluminium).

La plaque conductrice peut avoir une épaisseur comprise entre 0,1 mm et 0,5 mm, par exemple de 0,3 mm ou environ 0,3 mm. Ainsi, la plaque conductrice peut présenter une faible capacité calorifique sans compromettre sa rigidité. Une telle épaisseur de plaque conductrice permet un bon compromis entre le nombre de capteurs de températures mesurant la température de la plaque conductrice et la rigidité de la plaque conductrice.

Le dispositif peut comprendre une unité de commande électronique qui est reçue dans le boitier. L'unité de commande électronique est notamment configurée pour acquérir l'information de température mesurée par le ou les capteurs de température.

En particulier, une majorité de la pluralité de capteurs de température peut être située sur une portion inférieure de la plaque conductrice, la portion inférieure de la plaque conductrice étant destinée à être orientée vers le fond des toilettes. Ainsi, si le jet d'urine arrive en contact de la portion supérieure de la plaque, le ruissellement du liquide sur la face libre de la plaque peut être exploité pour la mesure de température. En effet, le ruissellement augmente la durée durant laquelle l'urine est au contact de la plaque conductrice, ce qui permet d'améliorer les transferts thermiques depuis le liquide vers la plaque conductrice. De plus, le dispositif, (par exemple l'unité de commande électronique) peut être configuré pour définir comme température finale de l'urine la température maximale parmi les températures déterminées par la pluralité de capteurs de température.

Chaque capteur de température peut être une thermistance. De tels capteurs sont peu sensibles aux perturbations ambiantes, nécessitent un conditionnement du signal peu complexe et peuvent couvrir une large gamme de températures. La tension aux bornes de la thermistance varie en fonction de la température (car sa résistance varie en fonction de la température).

Alternativement, chaque capteur de température pourrait être un capteur de température à semi-conducteur. De tels capteurs présentent une bonne robustesse et une bonne précision tout en étant relativement peu coûteux. Alternativement encore, chaque capteur de température peut être un capteur infrarouge thermopile. De la même façon, ces capteurs de température ont une tension à leurs bornes qui varie en fonction de la température.

Chaque capteur de température peut avoir une constante de temps inférieure à une seconde, par exemple inférieure à 0,2 secondes. Ainsi, chaque capteur de température peut enregistrer rapidement l'évolution de la température, ce qui rend le dispositif compatible avec la courte durée d'un jet d'urine.

Chaque capteur de température peut comprendre une face plane au contact de la plaque conductrice. En effet, la face plane favorise le transfert de chaleur depuis la plaque conductrice vers chaque capteur de température. En outre, la face plane permet un positionnement et un maintien plus aisé de chaque capteur de température sur la plaque conductrice.

Chaque capteur de température peut être attaché à une face arrière de la plaque conductrice (opposée à la face libre). En particulier, chaque capteur de température peut être collé sur la face arrière de la plaque conductrice à l'aide d'une colle conductrice de chaleur, par exemple de la colle conductrice de chaleur est une colle à polymérisation par Ultra-Violet (UV) ou une pâte thermique durcissante. Une telle colle conductrice permet de fixer le ou les capteurs de température sur la plaque conductrice sans compromettre le transfert de chaleur depuis la plaque conductrice vers le ou les capteurs de température. On note que l'utilisation de la pâte thermique permet une bonne conduction de chaleur depuis la plaque conductrice vers le ou les capteurs de températures. En variante, l'utilisation de la colle UV permet d'améliorer le maintien du ou des capteurs de températures sur la plaque conductrice.

Le dispositif d'analyse peut comprendre une pluralité de capteurs de température, et un même volume de colle conductrice peut être disposé entre chaque capteur de température et la plaque conductrice de chaleur. Ainsi, le temps de réponse à une évolution de température de la plaque est le même pour l'ensemble des capteurs de température. Les temps de réponse sont homogènes pour l'ensemble des capteurs de températures.

Le dispositif peut comprendre au moins une unité de traitement, configurée pour déterminer une donnée de température à partir de l'information de température mesurée par la pluralité de capteurs de température. L'unité de traitement permet de traiter les mesures de température de chaque capteur de température pour en déduire la température de l'urine. En particulier, chaque unité de traitement peut intégrer un capteur de température.

Comme indiqué auparavant, le dispositif peut comprendre une unité de commande électronique, configurée pour générer la donnée de température d'urine à partir des mesures de température obtenues par le ou les capteurs. L'unité de commande électronique peut comprendre la ou les unités de traitement. L'unité de commande électronique peut en outre comprendre un module de communication sans fil, configuré pour transmettre la donnée de température d'urine via un réseau sans fil (WiFi, Bluetooth, réseau cellulaire, etc.).

L'unité de commande électronique peut être reçue dans le boitier.

Le boîtier peut être en matériau isolant, par exemple en plastique, de type plastique non conducteur de chaleur (c'est-à-dire non chargé d'éléments conducteurs). Ainsi, les transferts thermiques entre la plaque conductrice et le boîtier sont réduits. L'influence du boîtier sur l'information de température mesurée par chaque capteur de température est ainsi limitée.

Le boîtier peut définir une ouverture recevant la plaque conductrice de chaleur. L'ouverture permet de réduire la surface de contact entre la plaque conductrice et le boîtier. On évite ainsi de créer des ponts thermiques entre la plaque conductrice et le boîtier.

Le boîtier peut comprendre une structure de support de la plaque conductrice. La plaque conductrice peut être supportée par la structure de support. Ainsi, la rigidité de la plaque conductrice peut être augmentée.

La plaque conductrice peut être bombée vers l'extérieur du boîtier. Le dispositif peut prendre alors une forme de galet circulaire. La forme de galet circulaire permet un bon ruissellement d'urine sur la surface extérieure du dispositif. La forme de galet circulaire est également esthétique et discrète.

Le boîtier peut présenter un témoin lumineux, qui comprend par exemple au moins une diode électroluminescente (LED), configuré pour afficher des informations à l'utilisateur. Des informations telles qu'un niveau de charge d'une batterie, ou une indication qu'une mesure de température est en cours, peuvent être facilement transmises à l'utilisateur ou l'utilisatrice (pour des questions de lisibilité, la suite de la description se référera à un utilisateur).

Le dispositif peut comprendre un moyen de communication (par exemple sans-fil) avec un dispositif de communication associé à l'utilisateur et/ou un serveur distant. L'analyse effectuée par le dispositif peut être transmise à l'utilisateur, notamment pour effectuer un suivi de la température basale. Le suivi de la température basale peut permettre le suivi de la fertilité ou pour détecter l'occurrence d'un épisode fiévreux.

Le dispositif peut être installé dans les toilettes par coopération avec un élément de fixation installé dans ou sur les toilettes. Le dispositif peut ainsi comprendre une attache coopérant avec l'élément de fixation. Par exemple, l'attache peut comprendre une fixation magnétique configurée pour coopérer avec au moins un aimant de l'élément de fixation. Par exemple, l'attache peut comprendre une fixation mécanique configurée pour coopérer avec un engagement mécanique de l'élément de fixation. Ainsi, le dispositif peut facilement être retiré des toilettes, par exemple pour recharger une batterie du dispositif. Alternativement, l'attache du dispositif peut être installé dans les toilettes sans coopération avec un élément de fixation spécifiquement installé dans ou sur les toilettes. Par exemple, l'attache peut être un crochet solidaire du boîtier et configuré pour s'engager avec un rebord de la cuvette (par-dessus ou par-dessous).

Selon un autre aspect, il est proposé un procédé de mesure de la température d'urine mis en oeuvre par le dispositif décrit ci-avant, comprenant :

- mesurer au moins une information de température à l'aide de la pluralité de capteurs de température lorsqu'un jet d'urine est reçu sur la face avant de la plaque conductrice ;
- déterminer une donnée de température de l'urine à partir de l'au moins une information de température.

Déterminer la donnée de température de l'urine peut comprendre réaliser une régression à deux exponentielles sur l'information de température et choisir la valeur limite de la régression comme la donnée de température de l'urine.

Le dispositif comprend une pluralité de capteurs de température, chaque capteur de température permettant de déterminer une donnée de température de l'urine, et le procédé peut comprendre : choisir la température maximale parmi les données de températures comme donnée de température finale du jet d'urine.

Le procédé peut comprendre envoyer la donnée de température vers un appareil à proximité et/ou un serveur distant.

Selon encore un autre aspect, il est proposé un procédé de suivi d'une période de fertilité comprenant :

- mettre en oeuvre le procédé de mesure de la température de l'urine,
- comparer la donnée de la température de l'urine à des données de températures d'urine antérieures ;
- détecter des variations entre les données de températures pour déterminer une période du cycle menstruel.

Brève **description des dessins**

D'autres caractéristiques, détails et avantages apparaîtront à la lecture de la description détaillée ci-après, et à l'analyse des dessins annexés, sur lesquels :

Fig. 1

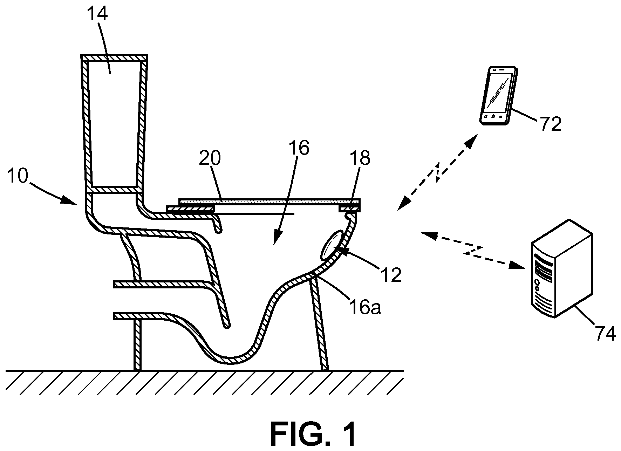

[Fig. 1] illustre schématiquement un dispositif d'analyse d'urine monté dans des toilettes selon un mode de réalisation.

**Fig. 2**

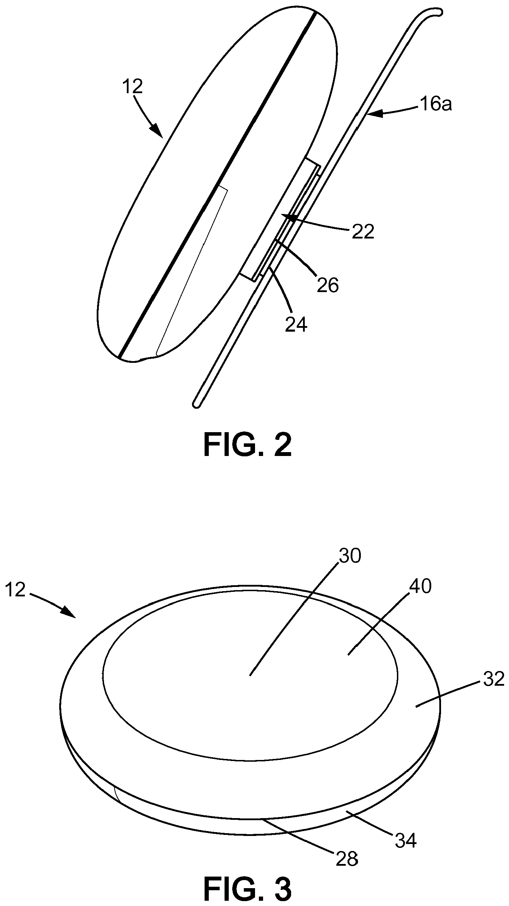

[Fig. 2] illustre schématiquement un détail de la figure 1 selon un mode de réalisation.

**Fig. 3**
[Fig. 3] illustre schématiquement une vue en perspective du dispositif d'analyse d'urine de la figure 1 selon un mode de réalisation.

**Fig. 4**

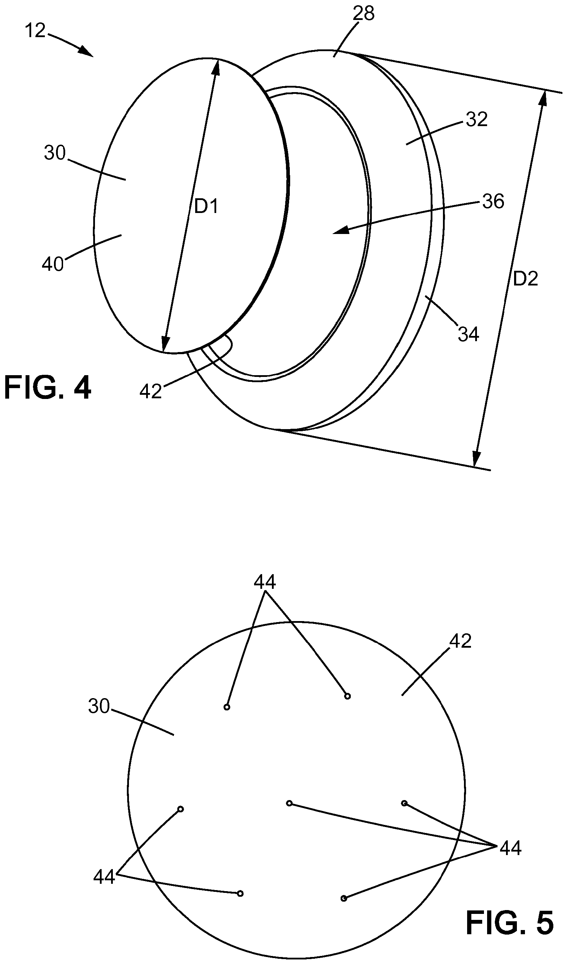

[Fig. 4] illustre schématiquement une vue éclatée du dispositif d'analyse d'urine de la figure 1 selon un mode de réalisation.

**Fig. 5**
[Fig. 5] illustre schématiquement une vue d'une face arrière d'une plaque conductrice et de capteurs de températures pouvant être mis en oeuvre dans le dispositif d'analyse d'urine de la figure 1 selon un mode de réalisation.

**Fig. 6**

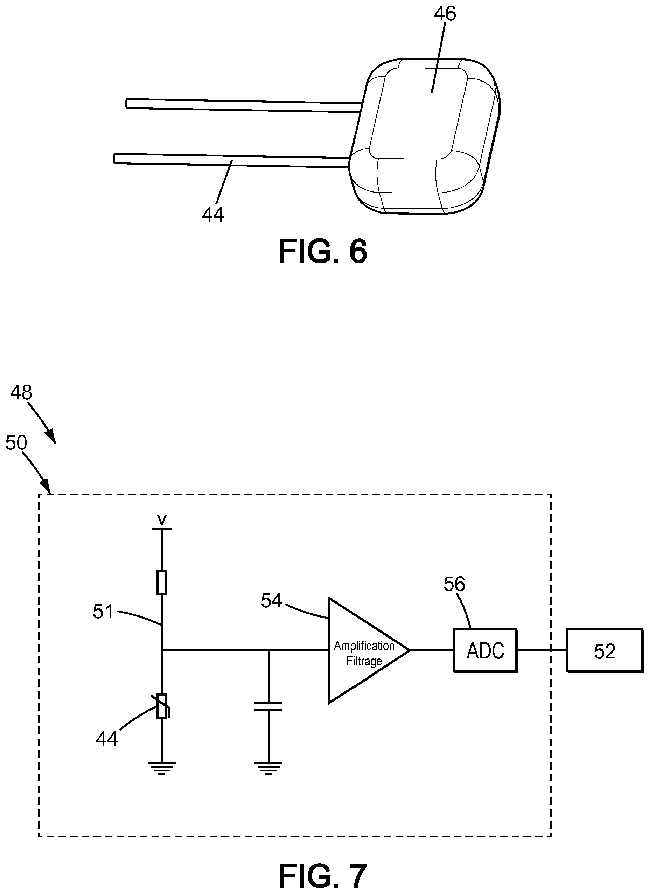

[Fig. 6] illustre schématiquement un capteur de température pouvant être mis en oeuvre dans le dispositif d'analyse d'urine de la figure 1 selon un mode de réalisation.

**Fig. 7**
[Fig. 7] illustre schématiquement une unité de traitement intégrant le capteur de température de la figure 6 selon un mode de réalisation.

**Fig. 8**

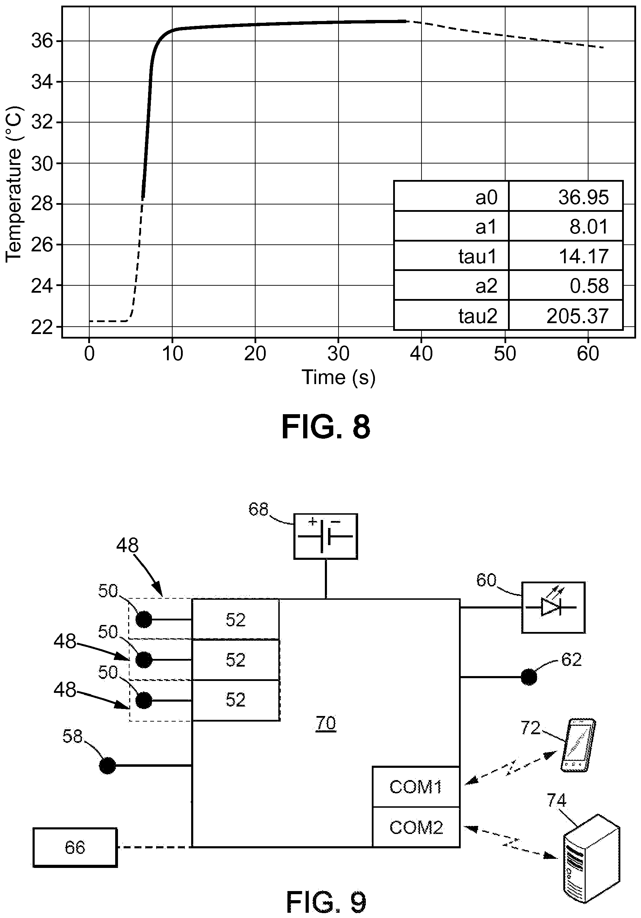

[Fig. 8] illustre un exemple d'évolution de température mesuré par l'unité de traitement de la figure 7 selon un mode de réalisation.

Fig. 9
[Fig. 9] illustre schématiquement des composants électroniques pouvant être mis en oeuvre dans le dispositif d'analyse d'urine de la figure 1 selon un mode de réalisation.

**Fig. 10**

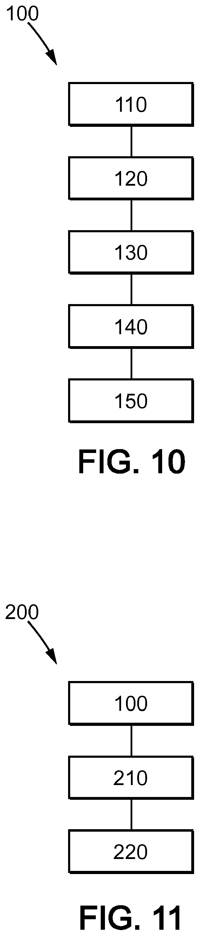

[Fig. 10] illustre une méthode mise en oeuvre par le dispositif d'analyse d'urine de la figure 1.

**Fig. 11**
[Fig. 11] illustre une méthode exploitant le dispositif d'analyse d'urine de la figure 1.

**Fig. 12**

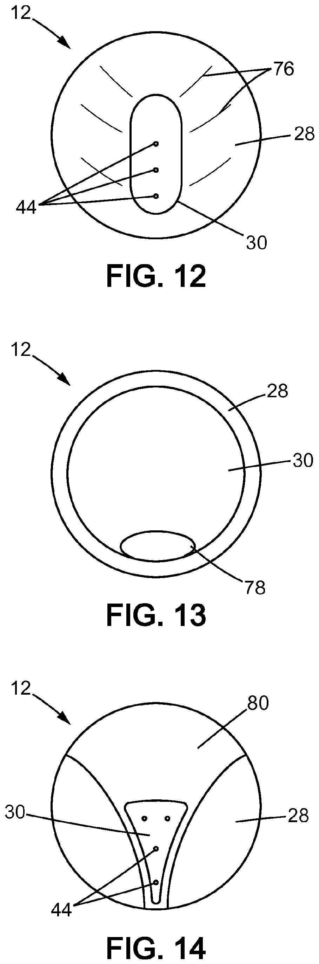

[Fig. 12] illustre schématiquement une vue de face d'un dispositif d'analyse d'urine selon un autre mode de réalisation.

**Fig. 13**
[Fig. 13] illustre schématiquement une vue de face d'un dispositif d'analyse d'urine selon un autre mode de réalisation.

**Fig. 14**

[Fig. 14] illustre schématiquement une vue de face d'un dispositif d'analyse d'urine selon un autre mode de réalisation.

## Description des modes de réalisation

La figure 1 illustre des toilettes 10 équipées d'un dispositif d'analyse d'urine 12.

De manière connue, les toilettes 10 comportent un réservoir d'eau 14, une cuvette 16, un siège 18 et un couvercle 20. Le dispositif d'analyse d'urine 12 est agencé sur une paroi interne 16a de la cuvette 16 des toilettes 10. Le dispositif d'analyse d'urine 12 est entièrement reçu dans les toilettes 10. Le dispositif d'analyse d'urine 12 est discret.

Comme illustré à la figure 2, le dispositif d'analyse d'urine 12 est positionné sur la paroi interne 16a des toilettes 10 par un élément de fixation 22. L'élément de fixation 22 permet de facilement retirer le dispositif d'analyse d'urine 12 des toilettes 10, par exemple pour recharger **une batterie 68** du dispositif d'analyse d'urine 12. L'élément de fixation 22 peut rester positionné sur les toilettes 10, alors que le dispositif d'analyse d'urine 12 peut être retiré.

L'élément de fixation 22 comprend ici une ventouse 24 et des aimants 26. La ventouse 24 peut adhérer à la paroi interne 16a des toilettes 10. Les aimants 26 sont destinés à coopérer avec une attache, comprenant ici des aimants ou des pièces magnétiques, du dispositif d'analyse d'urine 12. Alternativement, un couplage mécanique de type crochet peut faire coopérer l'élément de fixation 22 et le dispositif d'analyse d'urine 12. Le positionnement de l'élément de fixation 22 peut être choisi de sorte que le dispositif d'analyse d'urine 12 est sur une trajectoire du jet d'urine sécrété par un ou une utilisatrice. Le dispositif d'analyse d'urine 12 peut être positionné de manière à être sur la trajectoire du jet d'urine de l'utilisateur quel que soit son sexe.

En variante, pour l'adhésion à la paroi interne 16a des toilettes 10, l'élément de fixation 22 peut comprendre un adhésif, notamment un autocollant. Toujours en variante, l'élément de fixation 22 peut comprendre un crochet configuré pour s'engager avec un rebord de la cuvette 16.

Alternativement, le dispositif d'analyse d'urine 12 est positionné sur la paroi interne 16a des toilettes 10 sans utiliser d'élément de fixation dédié. L'attache du dispositif seule peut permettre un tel positionnement. L'attache du dispositif peut être un crochet configuré pour s'engager avec un rebord des toilettes 10. Dans cette alternative, lorsque le dispositif d'analyse d'urine 12 est retiré des toilettes 10, ces derniers sont à nouveau en condition d'origine. A l'inverse, lorsque l'élément de fixation 22 est utilisé, ce dernier peut demeurer en place dans les toilettes 10 lorsque le dispositif d'analyse d'urine 12 est retiré des toilettes 10.

Dans l'exemple illustré, l'élément de fixation 22 se loge dans un logement prévu sur le dispositif d'analyse d'urine 12. Le logement peut être prévu au niveau de connecteurs de charge de la batterie 68 du dispositif d'analyse d'urine 12. Ainsi, les connecteurs de charge peuvent être protégés de l'eau des toilettes 10.

L'élément de fixation 22 peut également comprendre des aides mécaniques. Les aides mécaniques peuvent par exemple être des indentations. Les aides mécaniques peuvent faciliter le positionnement du dispositif d'analyse d'urine 12 lors de son insertion dans les toilettes 10. La bonne position du dispositif d'analyse d'urine 12 dans les toilettes 10 assure le bon fonctionnement du dispositif d'analyse d'urine 12.

Par ailleurs, comme visible à la figure 2, le dispositif d'analyse d'urine 12 a ici une forme de galet circulaire. Le dispositif d'analyse d'urine 12 a ainsi une forme permettant un ruissellement d'urine sur les surfaces extérieures du dispositif d'analyse d'urine 12. Le temps de contact entre le dispositif d'analyse d'urine 12 et l'urine sécrétée par un utilisateur ou une utilisatrice dans les toilettes 10 est amélioré. L'utilisateur n'a pas besoin de « viser » pour s'assurer qu'un jet d'urine vienne au contact du dispositif d'analyse d'urine 12. La forme de galet circulaire donne également un aspect discret et esthétique au dispositif d'analyse d'urine 12.

Comme visible sur les figures 3 et 4, le dispositif d'analyse d'urine 12 comprend un boîtier 28 et une plaque conductrice de chaleur 30, montée sur le boîtier ou intégrée au boîtier.

### a) Plaque conductrice

La plaque conductrice 30 comprend une face libre 40 destinée à être orientée vers l'intérieur de la cuvette 16 des toilettes 10. Le jet d'urine peut entrer au contact du dispositif d'analyse d'urine 12 et ruisseler sur la face libre 40 plaque conductrice 30. La température du jet d'urine est transmise à la plaque conductrice 30. La plaque conductrice 30 agit comme un filtre passe-bas spatial permettant d'homogénéiser la température de l'urine sur toute la surface de la plaque conductrice 30.

La plaque conductrice 30 comprend également une face arrière 42, opposée à la face libre 40 et orientée vers le boîtier 28 ou vers l'intérieur du boîtier 28. La face arrière 42 permet de transmettre la température de l'urine relevée par la face libre 40 de la plaque conductrice 30 vers des composants électroniques contenus à l'intérieur du boîtier 28.

Comme visible sur les figures 3 et 4, la plaque conductrice 30 s'étend sur la majorité d'une face avant du boîtier 28. La plaque conductrice 30 occupe notamment une surface supérieure à 80% de la face avant du boîtier 28. La face avant du boîtier 28 désigne la face orientée vers l'intérieur de la cuvette 16 des toilettes 10 lorsque le dispositif d'analyse d'urine 12 est reçu dans les toilettes 10. La surface du dispositif d'analyse d'urine 12 disponible pour entrer au contact de l'urine est ainsi optimisée.

Par exemple, la plaque conductrice 30 peut avoir une surface supérieure à 3 500 mm$^2$, voire supérieure à 3 800 mm$^2$. Une telle surface permet un bon compromis entre la surface disponible pour relever la température de l'urine et la taille du dispositif d'analyse d'urine 12.

Ici, la plaque conductrice 30 est de forme sensiblement circulaire. Le diamètre D1 de la plaque conductrice 30 est compris entre 60 et 80 mm, par exemple 70 mm ou environ 70 mm. Par ailleurs, le diamètre D2 du boîtier 28, en forme de galet circulaire, est ici compris entre 75 et 110 mm.

Par ailleurs, la plaque conductrice 30 a une épaisseur comprise par exemple entre 0,1 mm et 0,5 mm. Dans un mode de réalisation, la plaque conductrice 30 a une épaisseur d'environ 0,3 mm. L'épaisseur de la plaque conductrice 30 correspond à la distance séparant la face libre 40 et la face arrière 42 de la plaque conductrice 30. Une telle épaisseur permet d'assurer une faible capacité calorifique de la plaque conductrice 30 pour un bon transfert thermique de l'urine vers le dispositif, tout en assurant que la plaque conductrice 30 présente une rigidité suffisante pour son intégration au dispositif d'analyse d'urine 12.

La plaque conductrice 30 peut également être bombée vers l'extérieur du boîtier 28. Une telle plaque conductrice 30 est compatible avec la forme de galet circulaire du dispositif d'analyse d'urine 12.

La plaque conductrice 30 est en métal conducteur de chaleur. Le métal formant la plaque conductrice 30 est choisi pour présenter de bonnes propriétés de conduction de chaleur. Dans un mode de réalisation, la plaque conductrice 30 est en aluminium. En effet, l'aluminium présente une bonne résistance à l'humidité et garde une bonne esthétique lorsque le dispositif d'analyse d'urine 12 est exposé à l'eau ou à l'urine. Alternativement, la plaque conductrice 30 pourrait être en un alliage de cuivre ou un acier inoxydable. Alternativement encore, la plaque conductrice 30 pourrait être en un plastique conducteur de chaleur. Un tel plastique peut comprendre un polymère contenant des éléments conducteurs de courant comme le graphite ou contenant des céramiques comme le nitrure d'aluminium.

La plaque conductrice 30 peut être fixée sur le boîtier 28 par collage, vissage ou tout autre moyen accessible à l'homme de l'art. Il peut être prévu un joint d'étanchéité entre la plaque conductrice 30 et le boîtier 28. Le joint d'étanchéité permet d'isoler l'intérieur du boîtier 28 de l'eau des toilettes 10.

b) Boîtier

Le boîtier 28 permet de loger les composants électroniques du dispositif d'analyse d'urine 12 et de les protéger du jet d'urine en les logeant dans un volume étanche (par étanche, on entend essentiellement étanche, c'est-à-dire étanche aux introductions non désirées d'urine dans le dispositif). Le volume étanche peut en outre être fermé par la plaque conductrice 30. Le boîtier 28 et la plaque conductrice 30 définissent ainsi le volume étanche, à l'intérieur duquel peut être logés les composants électroniques du dispositif d'analyse 12

Ici, le boîtier 28 comprend une coque avant 32 et une coque arrière 34. La coque avant 32 et la coque arrière 34 peuvent par exemple être reliées par vissage. Un joint d'étanchéité peut être prévu entre la coque avant 32 et la coque arrière 34. Les composants électroniques contenus dans le boîtier 28 peuvent être protégés de l'eau des toilettes 10.

Le boîtier 28 est en un matériau isolant thermique. Les transferts thermiques depuis le boîtier 28 vers la plaque conductrice de chaleur 30 peuvent être réduits. L'influence du boîtier 28 sur la température de la plaque conductrice 30 est réduite. Par exemple, le boîtier 28 est en plastique, en particulier un plastique non-conducteur. Le boîtier 28 présente alors une bonne isolation thermique, tout en étant relativement peu coûteux et facilement fabricable, notamment par moulage par injection.

Comme visible à la figure 4, le boîtier 28 définit une ouverture 36 destinée à recevoir la plaque conductrice 30. L'ouverture 36 permet de réduire la surface de contact entre le boîtier 28 et la plaque conductrice 30, limitant encore les transferts thermiques entre le boîtier 28 et la plaque conductrice 30. L'influence de la température du boîtier 28 sur la température de la plaque conductrice 30 est réduite.

Par ailleurs, le boîtier 28 peut définir une structure de renfort. La structure de renfort peut par exemple prendre la forme de nervures s'étendant à l'intérieur du boîtier 28. La structure de renfort peut soutenir la plaque conductrice 30, de sorte à augmenter sa rigidité.

c) capteurs de température

Le dispositif d'analyse d'urine 12 comprend une pluralité de capteurs de température 44 reçu à l'intérieur du boîtier 28. Chaque capteur de température 44 permet d'obtenir localement une information de température de la plaque conductrice 30. L'information de température peut être une valeur de température, c'est à dire une mesure discrète, ou une mesure de l'évolution de la température dans le temps, c'est-à-dire une mesure continue ou quasi-continue. En particulier, l'information de température peut être obtenue en récupérant la tension aux bornes du capteur de température.

Après traitement, l'information de température peut permettre d'obtenir une donnée de température de l'urine. Le traitement sera décrit par la suite. Par localement, on entend que chaque capteur de température 44 permet de mesurer la température et/ou l'évolution de la température (en prenant une pluralité de mesures de températures) d'au moins une portion de la plaque conductrice 30, qui est une portion située au voisinage du capteur de température 44.

Chaque capteur de température 44 a une constante de temps inférieure à 1 seconde, de préférence inférieure à 0,2 secondes. La constante de temps caractérise la réponse du capteur de température 44 à un changement de température. Chaque capteur de température 44 peut ainsi relever une information de la température de la plaque conductrice 30 causée par un jet d'urine court. Chaque capteur de température 44 peut également relever une information de la température lorsque le contact de l'urine avec la plaque conductrice 30 est de courte durée.

Dans un mode de réalisation, chaque capteur de température 44 est une thermistance. En effet, les thermistances sont peu sensibles aux perturbations ambiantes, sont peu complexes à exploiter et peuvent couvrir une large gamme de températures. Ils sont également facilement accessibles. Alternativement, chaque capteur de température 44 pourrait être un capteur de température à semi-conducteur. De tels capteurs de température présentent une bonne robustesse et une bonne précision tout en étant relativement peu coûteux. Alternativement encore, chaque capteur de température 44 peut être un capteur infrarouge thermopile.

Certains capteurs (comme les thermistances, les capteurs de température à semi-conducteur et les capteurs infrarouge thermopile) permettent en outre de mesurer en continu la température, et, de ce fait, de mesurer l'évolution dans le temps (par exemple lors d'une miction) de la température.

Dans l'exemple illustré à la figure 5, le dispositif 12 comprend une pluralité de capteurs de température 44 (sept capteurs de température illustrés). Les capteurs de température 44 sont positionnés de façon à permettre de mesurer des informations de température à plusieurs endroits de la plaque conductrice 30. La pluralité de capteurs de température 44 permet d'augmenter la probabilité que l'un des capteurs de température se trouve au voisinage du point d'impact du jet d'urine sur la plaque conductrice 30. L'information de température mesurée par le capteur de température 44 proche du point d'impact peut alors servir à déterminer la donnée de température de l'urine avec plus de précision.

La figure 5 illustre un exemple d'agencement de la pluralité de capteurs de température 44 sur la face arrière 42 de la plaque conductrice 30. Les capteurs 44 sont ainsi protégés par le boîtier 28, c'est-à-dire qu'ils sont dans le volume étanche défini par le boîtier 28 et la plaque conductrice 30. Dans cet exemple, sept capteurs de températures 44 sont agencés sur la face arrière 42 de la plaque conductrice 30. Le nombre de capteurs de température 44 peut être choisi selon les dimensions et l'épaisseur de la plaque conductrice 30. Le nombre de capteurs de température 44 est adapté à la conductivité de la plaque conductrice 30 et à ses dimensions. Par exemple, la pluralité de capteurs de température comprend entre 2 et 20 capteurs de température, voire entre 3 et 10 ou même 5 et 10. La répartition des capteurs de température sur la plaque conductrice 30 peut être régulière (par exemple : tous les 60° selon un cercle autour du centre de la plaque, avec ou sans un capteur de température au niveau du centre ; par exemple tous les 90° avec ou sans capteur de température au niveau du centre) ou essentiellement régulière.

Ici, une majorité de la pluralité de capteurs de températures 44 se situe sur une portion inférieure de la plaque conductrice 30 (sur la figure 5, au moins quatre capteurs de température sur les sept sont sur la portion inférieure). La portion inférieure de la plaque conductrice 30 correspond à la portion de la plaque conductrice 30 orientée vers le fond de la cuvette 16 des toilettes 10 lorsque le dispositif d'analyse d'urine 12 est reçu dans les toilettes 10. Dès lors, les capteurs de températures 44 peuvent exploiter le ruissellement d'urine sur la face libre 32 de la plaque conductrice 30. Le ruissellement de l'urine augmente le temps de contact de la plaque conductrice 30 avec l'urine, améliorant ainsi le transfert de chaleur depuis l'urine vers la plaque conductrice 30. L'information de température mesurée par chaque capteur de température 44 peut tendre vers la température de l'urine.

La figure 6 illustre plus en détails un exemple de capteur de température 44. Tel qu'illustré, le capteur de température 44 comprend une face plane 46. La face plane 46 peut être au contact de la face arrière 42 de la plaque conductrice 30 pour améliorer le transfert de chaleur depuis la plaque conductrice 30 vers le capteur de température 44.

Chaque capteur de température 44 est collé sur la face arrière 34 de la plaque conductrice 30 à l'aide d'une colle conductrice de chaleur. La colle conductrice permet d'assurer un bon transfert de chaleur depuis la plaque conductrice 30 vers le capteur de température 44. La colle conductrice peut notamment être une colle polymérisée par ultraviolets (UV). La colle conductrice permet notamment un bon maintien mécanique de chaque capteur de température 44 sur la plaque conductrice 30. La colle conductrice peut également être une pâte thermique, notamment une pâte thermique durcissante. La pâte thermique permet d'améliorer encore la conduction de chaleur depuis la plaque conductrice 30 vers le ou les capteurs de températures 44.

Lorsque le dispositif 12 comprend une pluralité de capteurs de température 44, chaque capteur de température 44 est collé à l'aide d'un même volume de colle conductrice. Le même volume de colle assure que le transfert thermique est sensiblement identique d'un capteur de température 44 à l'autre.

### d) Circuit de conditionnement

Le dispositif 12 comprend en outre une ou plusieurs unités de traitement 48. Chaque capteur de température 44 est intégré à une unité de traitement 48. Comme visible à la figure 7, l'unité de traitement 48 comprend un circuit électronique 50 et une unité de calcul 52.

Plus précisément, le circuit électronique 50 comprend typiquement un pont diviseur de tension 51, un étage de filtrage et d'amplification 54 et un convertisseur analogique-numérique 56.

Le pont diviseur de tension 51 permet de linéariser un signal, typiquement un signal de tension, généré par le capteur de température 44. Le pont diviseur de tension 51 permet de s'affranchir de la relation non-linéaire entre le signal généré par le capteur 44 et la température. Il apparaît possible de calibrer le ou les capteurs de températures 44 à partir de deux points de mesure.

L'étage de filtrage et d'amplification 54 est relié à une sortie du pont diviseur de tension 51. Le filtrage peut notamment être un filtre passe-bas appliqué au signal de sortie du pont diviseur de tension 51. Le filtrage permet de réduire le bruit et les autres artefacts hautes fréquences qui peuvent se trouver dans le signal. L'amplification consiste à appliquer un gain au signal, permettant d'augmenter la sensibilité du circuit à l'évolution de température de la plaque conductrice 30. Ainsi, chaque capteur de température 44 peut mesurer une gamme de température de 14°C à 41°C, avec une résolution de 1 millième de degré.

Le convertisseur analogique-numérique 56 est relié à une sortie de l'étage de filtrage et d'amplification 54. Le signal numérique obtenu peut être traité par l'unité de calcul 58.

La figure 8 illustre un exemple d'évolution de température de la plaque conductrice 30 mesurée par le capteur de température 44 lorsque l'utilisateur a réalisé une miction. L'évolution de la température illustrée correspond au signal de sortie du circuit électronique 50. L'unité de calcul 52 est configurée pour déterminer la donnée de température de l'urine à partir de l'information de température obtenue par le capteur de température 44.

Dans un mode de réalisation permettant d'obtenir une donnée de température plus précise, l'unité de calcul 52 réalise une régression de deux exponentielles sur l'évolution de température pour déterminer analytiquement la donnée de température. En effet, comme visible à la figure 8, l'évolution temporelle de la température peut se décrire avec une combinaison de deux processus simultanés. Le premier processus est une augmentation rapide de la température liée à l'apport de chaleur sur la plaque conductrice 30 par l'urine. Le second processus est une montée plus lente de la température liée à l'augmentation de la température de l'air à l'intérieur du boîtier 28. Dès lors, la régression de deux exponentielles permet de prendre en compte les deux processus.

La régression à deux exponentielles réalisée sur l'évolution de température peut notamment être la suivante :

[Math. 1]

$$T = a_0 - a_1 * e^{-\frac{t}{\tau_1}} - a_2 * e^{-\frac{t}{\tau_2}}$$

La première exponentielle (indice 1) modélise les transferts thermiques entre l'urine et la plaque conductrice 30, et la deuxième exponentielle (indice 2) modélise les transferts thermiques entre l'air à l'intérieur du boîtier 28 et la plaque conductrice 30. La valeur limite de la courbe de régression est $a_0$. La valeur limite correspond à la donnée de température de l'urine mesurée par le capteur de température 44. La donnée de température est ainsi déterminée analytiquement par régression de l'évolution de la température mesurée par chaque capteur de température 44, en utilisant la valeur limite ao déterminée par la régression. Cela permet d'améliorer la qualité de la mesure, permettant notamment une meilleure précision et une optimisation des informations.

Alternativement, aucune régression peut n'être utilisée et la température maximale mesurée à l'aide des capteurs de température est considérée comme la donnée de température. La donnée de température est ainsi déterminée empiriquement.

Bien entendu, d'autres algorithmes accessibles à l'homme du métier peuvent être exploités par l'unité de calcul 56 pour déterminer la donnée de température de l'urine.

On note que lorsque le dispositif d'analyse d'urine 12 comprend une pluralité de capteurs de température 44, chaque unité de calcul 52 détermine une donnée de température de l'urine. Une température finale peut être choisie parmi les données de températures déterminées. Alternativement, la température finale pourrait correspondre à une moyenne des températures déterminées par chaque unité de calcul 52 (soit analytiquement soit empiriquement, comme décrit précédemment).

Ici, la température finale est choisie comme la température maximale parmi les données de températures déterminées. La température maximale peut correspondre à celle déterminée par le capteur de température 44 le plus proche du point d'impact du jet d'urine sur la plaque conductrice 30. Alors, la donnée de température finale choisie est la plus fidèle à la température de l'urine.

### d) Autres composants

En se tournant vers la figure 9, le dispositif d'analyse d'urine peut comprendre divers composants électroniques.

Le dispositif d'analyse d'urine 12 peut comprendre un capteur de température supplémentaire 58. Le capteur de température supplémentaire 58 peut être placé dans le boîtier 28 pour estimer la température de l'air dans le boîtier 28. Le capteur de température supplémentaire 58 peut ainsi rendre compte des transferts thermiques avec l'air dans le boîtier 28.

Le dispositif d'analyse d'urine 12 peut comprendre un témoin lumineux 60. Le témoin lumineux 60 peut par exemple comprendre une ou plusieurs diodes électroluminescentes (LED). Le témoin lumineux 60 peut être visible depuis l'extérieur du boîtier 28. Le témoin lumineux 60 peut permettre d'informer l'utilisateur d'un état du dispositif d'analyse d'urine 12. Par exemple, le témoin lumineux 60 peut indiquer un état de charge de la batterie 64, qu'une analyse est en cours ou qu'une analyse a été réalisée. Le témoin lumineux 60 peut par exemple s'allumer lorsqu'une température au-delà d'un seuil prédéterminé est détectée par le ou les capteurs de températures 44. Par exemple, le seuil prédéterminé peut être 35°C. Le seuil prédéterminé correspond à une situation où un jet d'urine est reçu sur la plaque conductrice 30.

Le dispositif d'analyse d'urine 12 peut comprendre un bouton 62. Le bouton 62 est placé à l'intérieur du boîtier 28. Le bouton 62 peut être accédé par l'utilisateur en dévissant la coque avant 32 par rapport à la coque arrière 34 du boîtier 28. Le bouton 62 peut par exemple permettre de réinitialiser le dispositif d'analyse d'urine 12 et/ou d'initier une association sans-fil du dispositif 12 avec un appareil à proximité, par exemple un smartphone.

Le dispositif d'analyse d'urine 12 peut également comprendre un ensemble de test 66. L'ensemble de test 66 peut par exemple permettre de détecter la concentration d'un ou plusieurs composés de l'urine. Dans ce cas, le boîtier 28 présente un orifice de collecte pour collecter un échantillon d'urine. L'ensemble de test 68 peut acheminer l'échantillon d'urine collecté de l'orifice de collecte pour réaliser une analyse.

Par exemple, l'ensemble de test 66 peut réaliser une analyse par colorimétrie sur l'échantillon d'urine. L'échantillon d'urine peut être injecté sur une bandelette de test. La bandelette de test est ensuite analysée par un système optique.

En outre, le dispositif d'analyse d'urine 12 est alimenté par la batterie 68, notamment de type lithium ion. La batterie 68 est agencée dans le boîtier 28 pour alimenter les composants électroniques du dispositif d'analyse d'urine 12. La batterie 68 a par exemple une capacité de 1 080 mAH. Une telle capacité est un bon compromis entre l'encombrement de la batterie 68 dans le boîtier 28 et son autonomie.

La batterie 68 comprend des connecteurs de charge. Les connecteurs de charge peuvent être accessibles depuis l'extérieur du boîtier 28 pour permettre une recharge de la batterie. Par exemple, le dispositif d'analyse d'urine 12 peut être placé sur un socle pour relier les connecteurs de charge à une source d'énergie.

### e) Unité de commande électronique

Comme visible à la figure 9, le dispositif d'analyse d'urine 12 comprend une unité de commande électronique 70. L'unité de commande électronique 70 est reçue dans le boîtier 28.

L'unité de commande électronique 70 est reliée au circuit électronique 50 de chaque unité de traitement 48. En outre, l'unité de commande 70 intègre l'unité de calcul 52 de chaque unité de traitement 48. Ainsi, l'unité de commande 70 acquiert les informations de températures mesurées par chaque capteur de température 44. L'unité de commande 70 traite chaque information de température pour déterminer la donnée de température de l'urine, par exemple en mettant en oeuvre les différents traitements (régression, sélection de la valeur de température maximale, etc.). Lorsque le dispositif 12 comprend une pluralité de capteurs de température 44, l'unité de commande 70 peut déterminer la donnée de température finale de l'urine. L'unité de commande 70 permet ainsi de générer une donnée de température de l'urine à partir des mesures de température effectuées par les capteurs de température 44.

L'unité de commande 70 est également reliée aux autres composants électroniques du dispositif d'analyse d'urine 12. L'unité de commande 70 peut coopérer avec les autres composants électroniques.

En outre, l'unité de commande électronique 70 comprend au moins un module de communication sans fil, configuré pour transmettre à distance au moins la donnée de température.

L'unité de commande électronique 70 peut comprendre un premier module de communication sans-fil COM1 de courte portée. Le premier module de communication COM1 exploite un réseau local, par exemple de type Bluetooth, Low-Energy Bluetooth (BLE) ou Wi-fi. Le premier module de communication COM1 communique avec un dispositif à proximité 72 des toilettes 10. Le dispositif à proximité 72 peut par exemple être un smartphone, une montre connectée ou tout autre dispositif associé à l'utilisateur. Alternativement, le dispositif à proximité 72 peut être un appareil déporté monté à proximité des toilettes 10. Un tel appareil déporté peut par exemple comprendre un bouton et un affichage.

Le premier module de communication COM1 peut recevoir une commande d'analyse depuis le dispositif à proximité 72. La commande d'analyse informe le dispositif d'analyse d'urine 12 que l'utilisateur souhaite réaliser une analyse. L'analyse est notamment une mesure de température de l'urine, mais pourrait également être toute autre type d'analyse pouvant être mise en oeuvre par le dispositif d'analyse d'urine 12.

Le premier module de communication COM1 peut également recevoir une identification de l'utilisateur depuis le dispositif à proximité 72. L'identification peut permettre au dispositif d'analyse d'urine 12 de distinguer parmi différents utilisateurs utilisant les toilettes 10. L'analyse est alors propre à l'utilisateur.

Le premier module de communication COM1 peut également envoyer un résultat d'analyse vers le dispositif à proximité 72. Le résultat d'analyse comprend notamment la donnée de température de l'urine, mais pourrait également être tout autre résultat pouvant être déterminé par le dispositif d'analyse d'urine 12. L'utilisateur peut accéder au résultat immédiatement après une analyse.

L'unité de commande électronique 70 peut comprendre un deuxième module de communication sans-fil COM2. Le deuxième module de communication peut être prévu en complément ou en substitution du premier module de communication COM1. Le deuxième module de communication COM2 exploite le réseau cellulaire, par exemple GSM, 3G, 4G(-LTE), LTE-M, 5G ou tout réseau cellulaire dédié aux objets connectés. Le deuxième module de communication COM2 donne au dispositif d'analyse d'urine 12 une interface de communication vers un serveur distant 74.

Le deuxième module de communication COM2 peut envoyer le résultat d'analyse vers le serveur distant 74. Le résultat peut être envoyé directement, sans transiter par un dispositif de proximité 72 comme décrit précédemment. Le résultat peut ensuite être rendu accessible à l'utilisateur depuis tout appareil adapté à se connecter au serveur 74, par exemple le smartphone de l'utilisateur ou un ordinateur. Une pluralité de résultats peut être stockée par le serveur 74, permettant d'effectuer un suivi d'une pluralité d'analyses.

L'unité de commande 70 comprend en outre une mémoire MEM. La mémoire MEM peut stocker une pluralité de résultats d'analyse. La pluralité de résultats peut être envoyée périodiquement vers le serveur 74, par exemple une fois par semaine. La fréquence d'utilisation du deuxième module de communication COM2 peut être réduite, réduisant la consommation d'énergie de l'unité de commande 70.

De préférence, l'unité de commande 70 est construite par un système sur puce. Une première puce Bluetooth Low Energy (BLE) peut prendre en charge la commande des composants électroniques du dispositif d'analyse d'urine 12. La première puce BLE peut également prendre en charge la communication avec le dispositif à proximité 72. Une deuxième puce Wifi peut prendre en charge l'échange de données avec le serveur distant 74. Ainsi, le deuxième système sur puce peut être éteint lorsqu'une communication avec le serveur 74 n'est pas utilisé. La consommation d'énergie de l'unité de commande 70 est améliorée, et la consommation de la batterie 68 s'en trouve réduite.

L'unité de commande 70 peut également être divisée en différents circuits imprimés. Chaque circuit imprimé peut commander différents composants électroniques du dispositif d'analyse d'urine 12. Un circuit principal peut assurer la coopération entre les différents circuits imprimés. Une telle construction permet une flexibilité quant à la construction de l'unité de commande électronique 70 et son intégration dans le boîtier 28.

f) Aspect de procédé

On décrit par la suite un procédé 100 mis en oeuvre par le dispositif d'analyse d'urine 12, en particulier l'unité de commande 70 du dispositif d'analyse d'urine 12, en référence à la figure 10.

Dans une première étape 110, le dispositif d'analyse d'urine 12 initialise une analyse. L'initialisation d'une analyse peut notamment comprendre l'activation du ou des capteurs de température 44. Alternativement, le ou les capteurs de températures 44 peuvent continuellement mesurer l'évolution de la température de la plaque conductrice 30, et le l'initialisation d'une analyse comprend commencer un enregistrement de la mesure.

Le dispositif 12 peut initialiser une analyse en réponse à une requête de l'utilisateur via le dispositif à proximité 72. L'utilisateur peut par exemple lancer une commande depuis le smartphone ou appuyer sur un bouton de l'appareil déporté.

Le dispositif 12 peut initialiser une analyse en réponse à une détection par le dispositif d'analyse d'urine 12 du dispositif à proximité 72, porté par l'utilisateur. Par exemple, la détection peut inclure une détection que le dispositif de proximité 72 est à une distance du dispositif d'analyse d'urine 12 qui est inférieure à un seuil (par exemple 2m), ou inférieure à un seuil pendant au moins une durée prédéterminée. L'analyse est alors lancée automatiquement, sans action de la part de l'utilisateur.

Le dispositif 12 peut initialiser une analyse en réponse à une détection d'un jet d'urine. Par exemple, la détection peut inclure une détection le ou les capteurs de température 44 que la température mesurée dépasse un seuil prédéterminé, par exemple 35°C. L'analyse peut alors être lancée sans communication avec le dispositif à proximité 72.

Dans une deuxième étape 120, le dispositif 12 réalise la mesure de la température pour générer une donnée de température. Chaque capteur de température 44 mesure localement une information de température de la plaque conductrice 30 pendant que l'utilisateur réalise une miction. Par exemple, l'information est une évolution de la température dans le temps. Alors, la température peut être mesurée jusqu'à ce que la température de la plaque conductrice 30 retombe en dessous du seuil. L'évolution de température peut également être mesurée pour une durée déterminée, par exemple entre 2 et 4 minutes.

L'information de température mesurée par chaque capteur 44 peut être linéarisée pour faciliter la calibration du ou des capteurs de température. L'information de température peut également être amplifiée pour améliorer la sensibilité de la mesure et filtrée pour réduire le bruit de la mesure. Les mesures de température peuvent en outre être converties en un signal numérique pour permettre son traitement.

Dans une troisième étape 130, le dispositif 12 utilise les mesures de température de la plaque pour déterminer une donnée de température de l'urine. En particulier, chaque information de température peut être utilisée pour déterminer une donnée de température de l'urine. La température de l'urine peut par exemple être déterminée en appliquant une régression à deux exponentielles sur l'évolution de température. La valeur limite de la courbe de régression est alors choisie comme la donnée de température de l'urine.

Lorsque le dispositif d'analyse d'urine 12 comprend une pluralité de capteurs de température 44, les données de température d'urine estimées pour chaque capteur de température 44 peuvent être comparées. Plus particulièrement, la température maximale peut-être identifiée. La température maximale peut être choisie comme la donnée de température finale de l'urine.

Selon une quatrième étape 140, le dispositif 12 transmet la donnée de température déterminée au dispositif à proximité 72. Le dispositif à proximité 72 peut afficher la donnée de température à l'utilisateur. Le dispositif à proximité 72 peut également afficher une courbe comprenant illustrant la donnée de température et des données de température mesurées antérieurement. L'utilisateur peut ainsi visualiser les variations de températures au cours du temps.

Selon une cinquième étape 150, complémentaire ou alternative à la quatrième étape 140, la donnée de température de l'urine peut être envoyée vers le serveur distant 74, typiquement sans transiter par le dispositif à proximité. Le serveur distant 74 peut stocker les mesures réalisées pour permettre à l'utilisateur d'y accéder.

Dans un exemple d'application, le dispositif d'analyse d'urine 12 peut permettre à un utilisateur d'identifier une fenêtre de fertilité. Ainsi, on décrit par la suite un procédé 200 de suivi de la période de fertilité, en référence à la figure 11.

Le procédé 200 comprend une première étape de mise en oeuvre du procédé 100 décrit ci-avant.

Dans une deuxième étape 210, la donnée de température est comparée à des données de température obtenues antérieurement. De préférence, une mesure de température est prise chaque jour, et les données de température antérieures correspondent aux mesures effectuées les jours précédant celui de l'obtention de la nouvelle donnée de température.

Dans une troisième étape 220, les variations entre les différentes données de températures sont identifiées. Les variations permettent d'identifier le stade du cycle menstruel de l'utilisatrice. Par exemple, une baisse de la température peut indiquer un début de période de fertilité et une augmentation de la température peut indiquer que l'ovulation a eu lieu.

En variante, le dispositif d'analyse d'urine 12 peut permettre d'identifier un épisode fiévreux. Dans ce cas, les variations entre les différentes données de températures peuvent détecter une augmentation anormale de la température basale.

g) Variantes

Le dispositif d'analyse d'urine 12 décrit ci-avant ne se limite pas aux seuls modes de réalisation décrits ci-avant, mais est, au contraire, susceptible de nombreuses variantes accessibles à l'homme de l'art.

Le dispositif d'analyse d'urine a été décrit en relation avec des toilettes 10, dites classique, comprenant un siège sur lequel l'utilisateur peut s'asseoir. Toutefois, le dispositif d'analyse d'urine peut être installé dans un urinoir.

La plaque conductrice 30 pourrait notamment être texturée de sorte à canaliser l'urine vers le ou les capteurs de température 44. Cette solution vise à améliorer la précision de l'évolution de température mesurée par le ou les capteurs de température 44.

Dans l'exemple de la figure 12, une pluralité de nervures 76 s'étendent depuis une périphérie de la plaque conductrice 30 vers une partie centrale de la plaque conductrice 30. Le ou les capteurs de températures 44 pourraient alors être prévus au niveau de la partie centrale de la plaque conductrice 30.

Alternativement, dans l'exemple de la figure 13, la partie inférieure de la plaque conductrice 30 présente une saillie 78. La saillie 78 permet de retenir l'urine ruisselant sur la face libre 40 de la plaque conductrice 30. Le temps de contact entre l'urine et la plaque conductrice 30 est augmenté.

Par ailleurs, dans l'exemple de la figure 14, le boîtier 28 forme un renfoncement 80 dans lequel est reçue la plaque conductrice 30. Le renfoncement 80 peut former un canal s'étendant depuis une partie supérieure du boîtier 28 vers une partie inférieure du boîtier 28. La plaque conductrice 30 peut s'étendre au niveau de la partie inférieure, de sorte que l'urine circulant dans le canal s'écoule sur la plaque conductrice 30.

Par ailleurs, le boîtier 28 et la plaque conductrice 30 pourraient être d'une seule pièce. L'ensemble de la surface extérieure du dispositif d'analyse d'urine 12 est alors en matériau conducteur de chaleur. Le transfert thermique entre l'urine et le dispositif d'analyse 12 peut être augmenté.

Dans le mode de réalisation où la plaque est en plastique conducteur, le procédé de fabrication peut impliquer d'obtenir un plastique du boîtier qui ne soit pas conducteur de chaleur et un plastique de la plaque conductrice qui soit conducteur de chaleur. A cette fin, la charge en élément conducteur peut être ajustée lors de la fabrication de la pièce. Typiquement, lors d'un procédé de fabrication de la pièce par moulage, une partie du moule correspondant au boîtier peut recevoir un simple polymère et une partie du moule correspondant à la plaque conductrice peut recevoir un polymère (par exemple le même que celui pour le boîtier) chargé en éléments conducteurs (voir infra). Un tel procédé permet de générer une pièce d'un seul tenant qui comportant, grâce à aux propriétés de ses matériaux des régions plus conductrices thermiquement que d'autres.

## Revendications

1. Dispositif d'analyse d'urine (12) configuré pour être entièrement positionné à l'intérieur de toilettes (10), le dispositif comprenant :

   - un boîtier (28),
   - une plaque conductrice de chaleur (30), montée sur le boîtier (28) et comprenant une face libre (40) destinée à recevoir un jet d'urine directement d'un utilisateur urinant dans les toilettes (10),

   le dispositif étant **caractérisé en ce qu'**il comprend en outre :

   - une pluralité de capteurs de température (44), chaque capteur (44) de température étant configuré pour mesurer localement une information de température de la plaque conductrice (30).

2. Dispositif selon la revendication 1, dans lequel la plaque conductrice (30) a une surface supérieure à 3 500 mm$^2$, par exemple supérieure à 3 800 mm$^2$.

3. Dispositif selon la revendication 1 ou 2, dans lequel la plaque conductrice (30) est en métal conducteur de chaleur, par exemple la plaque conductrice (30) est en aluminium.

4. Dispositif selon l'une quelconque des revendications 1 à 3, dans lequel l'au moins un capteur de température (44) est positionné dans un volume défini par le boîtier (28) et la plaque conductrice de chaleur (30).

5. Dispositif selon l'une quelconque des revendications 1 à 4, dans lequel une majorité de la pluralité de capteurs de température (44) est située sur une portion inférieure de la plaque conductrice (30), la portion inférieure de la plaque conductrice (30) étant destinée à être orientée vers le fond des toilettes (10).

6. Dispositif selon l'une quelconque des revendications 1 à 5, configuré pour choisir, parmi les températures déterminées par la pluralité de capteurs de température (44), la température maximale comme donnée de température finale de l'urine.

7. Dispositif selon l'une quelconque des revendications 1 à 6, comprenant au moins une unité de traitement (48) configurée pour déterminer une donnée de température de l'urine à partir de l'information de température mesurée par le capteur de température (44).

8. Dispositif selon la revendication 7, dans lequel déterminer la donnée de température de l'urine comprend réaliser une régression à deux exponentielles sur l'information de température et choisir la valeur limite de la régression comme la donnée de température de l'urine.

9. Dispositif selon l'une quelconque des revendications 1 à 8, dans lequel le boîtier (28) définit une ouverture (36) recevant la plaque conductrice de chaleur (30).

10. Dispositif selon l'une quelconque des revendications 1 à 9, le dispositif (12) ayant une forme de galet circulaire, et/ou dans lequel la plaque conductrice (30) est bombée vers l'extérieur du boîtier (28).

11. Dispositif selon l'une quelconque des revendications 1 à 10, comprenant un moyen de communication sans-fil (50) avec un dispositif de communication (60) associé à l'utilisateur et/ou un serveur distant (64), pour transmettre au moins une donnée de température.

12. Procédé de mesure de la température d'urine mis en oeuvre par le dispositif selon l'une quelconque des revendications précédentes, comprenant :

   - mesurer au moins une information de température à l'aide de la pluralité de capteurs de température (44) lorsqu'un jet d'urine est reçu sur la face libre (40) de la plaque conductrice (30) ;
   - déterminer une donnée de température de l'urine à partir de l'information de température.

13. Procédé selon la revendication 12, dans lequel déterminer la donnée de température de l'urine comprend réaliser une régression à deux exponentielles sur l'information de température et choisir la valeur limite de la régression comme la donnée de température de l'urine.

14. Procédé selon l'une quelconque des revendications 12 ou 13, chaque capteur de température permettant de déterminer une donnée de température de l'urine, et le procédé comprend choisir la température maximale parmi les températures déterminées comme donnée de température finale de l'urine.

15. Procédé de suivi d'une période de fertilité comprenant :

   - mettre en oeuvre le procédé de mesure de température selon l'une des revendications 12 à 14 ;
   - comparer la donnée de température de l'urine à des données de températures d'urines antérieures ;
   - détecter des variations entre les données de températures pour déterminer une période du cycle menstruel.


**Patentansprüche**

1. Urinanalysevorrichtung (12), die so konfiguriert ist, dass sie vollständig innerhalb einer Toilette (10) positioniert werden kann, wobei die Vorrichtung umfasst:

   - ein Gehäuse (28),
   - eine wärmeleitende Platte (30), die an dem Gehäuse (28) angebracht ist und eine freie Seite (40) aufweist, die dazu bestimmt ist, einen Urinstrahl direkt von einem Benutzer aufzunehmen, der in die Toilette (10) uriniert,

   wobei die Vorrichtung **dadurch gekennzeichnet ist, dass** sie außerdem umfasst:

   - eine Vielzahl von Temperatursensoren (44), wobei jeder Temperatursensor (44) so konfiguriert ist, dass er lokal eine Temperaturinformation der leitfähigen Platte (30) misst.

2. Vorrichtung nach Anspruch 1, wobei die leitfähige Platte (30) eine Fläche von mehr als 3.500 mm$^2$, z. B. mehr als 3.800 mm$^2$, aufweist.

3. Vorrichtung nach Anspruch 1 oder 2, wobei die leitfähige Platte (30) aus einem wärmeleitenden Metall besteht, z. B. besteht die leitfähige Platte (30) aus Aluminium.

4. Vorrichtung nach einem der Ansprüche 1 bis 3, wobei der mindestens eine Temperatursensor (44) in einem Volumen positioniert ist, das durch das Gehäuse (28) und die wärmeleitende Platte (30) definiert ist.

5. Vorrichtung nach einem der Ansprüche 1 bis 4, bei der sich eine Mehrheit der mehreren Temperatursensoren (44) auf einem unteren Abschnitt der leitenden Platte (30) befindet, wobei der untere Abschnitt der leitenden Platte (30) dazu bestimmt ist, zum Boden der Toilette (10) zu weisen.

6. Vorrichtung nach einem der Ansprüche 1 bis 5, die so konfiguriert ist, dass sie aus den von der Vielzahl von Temperatursensoren (44) ermittelten Temperaturen die Höchsttemperatur als Endtemperaturangabe für den Urin auswählt.

7. Vorrichtung nach einem der Ansprüche 1 bis 6, mit mindestens einer Verarbeitungseinheit (48), die so konfiguriert ist, dass sie aus der vom Temperatursensor (44) gemessenen Temperaturinformation eine Temperaturangabe des Urins ermittelt.

8. Vorrichtung nach Anspruch 7, wobei das Bestimmen der Temperaturdaten des Urins das Durchführen einer zweifach exponentiellen Regression auf die Temperaturinformation und das Auswählen des Grenzwerts der Regression als die Temperaturdaten des Urins umfasst.

9. Vorrichtung nach einem der Ansprüche 1 bis 8, wobei das Gehäuse (28) eine Öffnung (36) definiert, die die wärmeleitende Platte (30) aufnimmt.

10. Vorrichtung nach einem der Ansprüche 1 bis 9, wobei die Vorrichtung (12) eine kreisförmige Rollenform aufweist, und/oder wobei die leitende Platte (30) aus dem Gehäuse (28) heraus gewölbt ist.

11. Vorrichtung nach einem der Ansprüche 1 bis 10, die ein Mittel zur drahtlosen Kommunikation (50) mit einem dem Benutzer zugeordneten Kommunikationsgerät (60) und/oder einem entfernten Server (64) umfasst, um mindestens ein Temperaturdatum zu übertragen.

12. Verfahren zur Messung der Temperatur von Urin, das von der Vorrichtung nach einem der vorhergehenden Ansprüche durchgeführt wird, umfassend:

    - Messen mindestens einer Temperaturinformation mit Hilfe der Vielzahl von Temperatursensoren (44), wenn ein Urinstrahl auf die freie Seite (40) der leitfähigen Platte (30) trifft;
    - Bestimmen einer Temperaturangabe des Urins aus der Temperaturinformation.

13. Verfahren nach Anspruch 12, wobei das Bestimmen der Temperaturdaten des Urins das Durchführen einer Zwei-Exponential-Regression auf die Temperaturinformation und das Auswählen des Grenzwerts der Regression als die Temperaturdaten des Urins umfasst.

14. Verfahren nach einem der Ansprüche 12 oder 13, wobei jeder Temperatursensor die Bestimmung von Temperaturdaten des Urins ermöglicht und das Verfahren die Auswahl der Höchsttemperatur aus den bestimmten Temperaturen als endgültige Temperaturdaten des Urins umfasst.

15. Verfahren zur Überwachung einer fruchtbaren Periode, umfassend:

    - Durchführen des Temperaturmessverfahrens nach einem der Ansprüche 12 bis 14;
    - Vergleichen der Temperaturdaten des Urins mit Temperaturdaten früherer Urine;
    - Erfassen von Abweichungen zwischen den Temperaturdaten, um eine Periode des Menstruationszyklus zu bestimmen.

**Claims**

1. A urinalysis device (12) configured to be positioned entirely within a toilet (10), the device comprising:

    - a housing (28),
    - a heat-conducting plate (30), mounted on the housing (28) and comprising a free face (40) intended to receive a stream of urine directly from a user urinating in the toilet (10),

the device being **characterised in that** it further comprises:

    - a plurality of temperature sensors (44), each temperature sensor (44) being configured to locally measure temperature information of the conductive plate (30).

2. The device according to claim 1, wherein the conductive plate (30) has a surface area greater than 3,500 $mm^2$, for example greater than 3,800 $mm^2$.

3. The device according to claim 1 or 2, wherein the conductive plate (30) is made of heat-conducting metal, for example the conductive plate (30) is made of aluminium.

4. The device according to any one of claims 1 to 3, in which the at least one temperature sensor (44) is positioned in a volume defined by the housing (28) and the heat-conducting plate (30).

5. The device according to any one of claims 1 to 4, wherein a majority of the plurality of temperature sensors (44) are located on a lower portion of the conductive plate (30), the lower portion of the conductive plate (30) being intended to be oriented towards the bottom of the toilet (10).

6. The device according to any one of claims 1 to 5, configured to select, from among the temperatures determined by the plurality of temperature sensors (44), the maximum temperature as the final temperature of the urine.

7. The device according to any one of claims 1 to 6, comprising at least one processing unit (48) configured to determine urine temperature data from the temperature information measured by the temperature sensor (44).

8. The device according to claim 7, wherein determining the urine temperature data comprises performing a two-exponential regression on the temperature information and selecting the limit value of the regression as the urine temperature data.

9. The device according to any one of claims 1 to 8, in which the housing (28) defines an opening (36) receiving the heat-conducting plate (30).

10. The device according to any one of claims 1 to 9, the device (12) being in the form of a circular roller, and/or in which the conductive plate (30) bulges outwards from the housing (28).

11. The device according to any one of claims 1 to 10, comprising wireless communication means (50) with a communication device (60) associated with the user and/or a remote server (64), for transmitting at least one temperature data item.

12. A method of measuring the temperature of urine implemented by the device according to any one of the preceding claims, comprising:

  - measuring at least one item of temperature information using the plurality of temperature sensors (44) when a stream of urine is received on the free face (40) of the conductive plate (30);
  - determining a temperature datum of the urine from the temperature information.

13. The method according to claim 12, wherein determining the urine temperature data comprises performing a two-exponential regression on the temperature information and selecting the limit value of the regression as the urine temperature data.

14. The method according to any one of claims 12 or 13, each temperature sensor making it possible to determine a temperature datum for the urine, and the method comprises selecting the maximum temperature from the temperatures determined as the final temperature datum for the urine.

15. A method of monitoring a fertile period comprising:

  - implementing the temperature measurement method according to one of claims 12 to 14;
  - comparing the urine temperature data with previous urine temperature data;
  - detecting variations between the temperature data to determine a period of the menstrual cycle.

**FIG. 1**

12

16a

22

26

24

## FIG. 2

12

30

40

32

28

34

## FIG. 3

FIG. 4

FIG. 5

**FIG. 6**

Amplification
Filtrage

ADC

**FIG. 7**

| | |
|---|---|
| a0 | 36.95 |
| a1 | 8.01 |
| tau1 | 14.17 |
| a2 | 0.58 |
| tau2 | 205.37 |

**FIG. 8**

**FIG. 9**

100

```
┌─────────────┐
│     110     │
└─────────────┘
       │
┌─────────────┐
│     120     │
└─────────────┘
       │
┌─────────────┐
│     130     │
└─────────────┘
       │
┌─────────────┐
│     140     │
└─────────────┘
       │
┌─────────────┐
│     150     │
└─────────────┘
```

## FIG. 10

200

```
┌─────────────┐
│     100     │
└─────────────┘
       │
┌─────────────┐
│     210     │
└─────────────┘
       │
┌─────────────┐
│     220     │
└─────────────┘
```

## FIG. 11

**FIG. 12**

**FIG. 13**

**FIG. 14**

**RÉFÉRENCES CITÉES DANS LA DESCRIPTION**

**Documents brevets cités dans la description**

- JP S63176536 B
- WO 2005092177 A